(19) Europäisches Patentamt
European Patent Office
Office européen des brevets

(11) Veröffentlichungsnummer: **0 198 382**
**B1**

# (12) EUROPÄISCHE PATENTSCHRIFT

(45) Veröffentlichungstag der Patentschrift: **20.09.89**

(21) Anmeldenummer: **86104721.5**

(22) Anmeldetag: **07.04.86**

(51) Int. Cl.⁴: **C07D 233/60**, C07D 213/82, C07D 213/83, C07D 239/26, C07D 241/12, C07D 249/08, A01N 43/40, A01N 43/50, A01N 43/54, A01N 43/60, A01N 43/653

## (54) Heterocyclische Amid-Derivate.

(30) Priorität: **13.04.85 DE 3513259**

(43) Veröffentlichungstag der Anmeldung:
**22.10.86 Patentblatt 86/43**

(45) Bekanntmachung des Hinweises auf die Patenterteilung:
**20.09.89 Patentblatt 89/38**

(84) Benannte Vertragsstaaten:
**AT BE CH DE FR GB IT LI NL SE**

(56) Entgegenhaltungen:
**EP-A- 0 024 017**
**EP-A- 0 076 030**
**EP-A- 0 121 344**
**DE-A- 3 102 590**
**DE-A- 3 339 644**
**US-A- 3 308 131**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Holmwood, Graham, Dr., Krutscheider Weg 105, D-5600 Wuppertal 11(DE)**
Erfinder: **Weissmüller, Joachim, Dr., Carl-Langhans-Strasse 53, D-4019 Monheim(DE)**
Erfinder: **Brandes, Wilhelm, Dr., Eichendorffstrasse 3, D-5653 Leichlingen 1(DE)**
Erfinder: **Reinecke, Paul, Dr., Steinstrasse 8, D-5090 Leverkusen 3(DE)**

EP 0 198 382 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue heterocyclische Amid-Derivate, mehrere Verfahren zu ihrer Herstellung und ihre Verwendung als Fungizide.

Es ist bekannt geworden, daß bestimmte Amid-Derivate, wie beispielsweise N-[2-(2,4,6-Trichlorphenoxy)-ethyl]-N-propyl-1H-imidazol-1-carboxamid (vgl. DE-A 2 429 523), und N-Trichlormethylmercaptotetra-hydrophthalimid (vgl. K.H. Büchel, Pflanzenschutz und Schädlingsbekämpfung, Seite 140, Georg Thieme Verlag Stuttgart, 1977) fungizide Eigenschaften aufweisen. Die Wirksamkeit dieser Verbindungen ist jedoch, insbesondere bei niedrigen Aufwandmengen und -konzentrationen, nicht immer ganz zufriedenstellend.

Ferner ist aus der DE-A 3 339 644 bekannt, daß sich phytopathogene Pilze auch mit Hilfe von solchen Heteroarylamiden bekämpfen lassen, die am Amidstickstoffatom einen gegebenenfalls substituierten Phenylalkylen-, Phenoxyalkylen- oder Phenylthioalkylen-Rest enthalten. Entsprechende Naphtyl-Verbindungen werden aber nicht offenbart.

Es wurden neue heterocyclische Amid-Derivate der allgmeinen Formel (I)

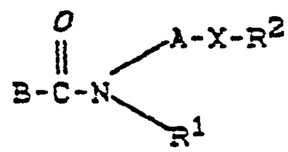

(I)

in welcher

$R^1$ für Alkyl mit 1 bis 12 Kohlenstoffatomen und für gegebenenfalls durch Fluor oder Chlor substituiertes Alkenyl und Alkinyl mit jeweils 3 bis 12 Kohlenstoffatomen steht, wobei die Mehrfachbindung jeweils nicht in α-Stellung zum Stickstoffatom stehen darf, sowie für Alkoxyalkyl oder Alkylthioalkyl mit jeweils 1 bis 6 Kohlenstoffatomen im Alkoxy- bzw. Alkylthioteil und 2 bis 6 Kohlenstoffatomen im Alkylteil steht;

$R^2$ für gegebenenfalls einfach bis dreifach gleich oder verschieden durch Alkyl mit 1 bis 4 Kohlenstoffatomen, Halogen und Halogenalkoxy mit 1 bis 4 Kohlenstoffatomen und 1 bis 5 gleichen oder verschiedenen Halogenatomen substituiertes Naphtyl, Tetrahydronaphtyl oder Indanyl steht;

A für eine geradkettige oder verzweigte Alkylenbrücke mit 1 bis 8 Kohlenstoffatomen steht, wenn X eine direkte Bindung bedeutet, bzw. für eine geradkettige oder verzweigte Alkylenbrücke mit 2 bis 8 Kohlenstoffatomen steht, wenn X Sauerstoff oder Schwefel bedeutet, wobei jedoch zwischen dem Stickstoffatom und dem Rest X mindestens 2 Kohlenstoffatome stehen müssen, oder für eine geradkettige oder verzweigte Alkylenbrücke mit 3 bis 8 Kohlenstoffatomen steht, wobei die Doppelbindung nicht in α-Stellung zum Stickstoffatom stehen darf;

X für Sauerstoff, Schwefel, sowie für eine direkte Bindung steht und

B für Imidazol-1-yl, Pyridin-3-yl, Pyrazin-2-yl, Pyrimidin-5-yl, 1,2,4-Triazol-1-yl sowie für 1-Methyl-imidazol-5-yl steht;

sowie deren Säureadditions-Salze und Metallsalz-Komplexe gefunden.

Weiterhin wurde gefunden, daß man die heterocyclischen Amid-Derivate der Formel (I)

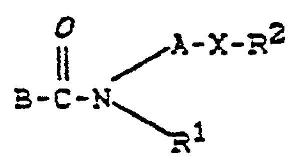

(I)

in welcher $R^1$, $R^2$, A, X und B die oben angegebenen Bedeutungen haben, sowie deren Säureadditions-Salze und Metallsalz-Komplexe erhält, wenn man

a) Amine der allgemeinen Formel (II)

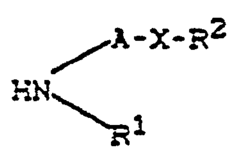

(II)

in welcher $R^1$, $R^2$, A und X die oben angegebenen Bedeutungen haben, mit Carbonyl-Verbindungen der allgemeinen Formel (IIIa)

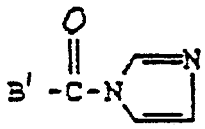

(IIIa)

in welcher $B^1$ für Imidazol-1-yl, Pyridin-3-yl, Pyrazin-2-yl, Pyrimidin-5-yl oder 1-Methylimidazol-5-yl steht, oder mit einer Carbonyl-Verbindung der allgemeinen Formel (IIIb)

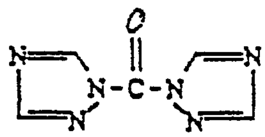

(IIIb)

in Gegenwart eins geeigneten inerten organischen Lösungsmittel umsetzt; oder

b) Amine der allgemeinen Formel (II)

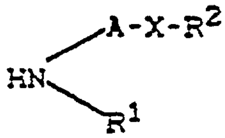

(II)

in welcher $R^1$, $R^2$, A und X die oben angegebenen Bedeutungen haben, mit Carbamoylchlorid-Verbindungen der Formel (IV)

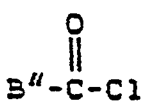

(IV)

in welcher B" für Pyridin-3-yl, Pyrazin-2-yl, Pyrimidin-5-yl, oder 1-Methylimidazol-5-yl steht, in Gegenwart eines geeigneten inerten organischen Lösungsmittels und in Gegenwart eines Säurebindemittels umsetzt; oder

c) Carbamoylchlorid-Derivate der allgemeinen Formel (V)

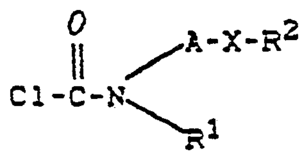

(V)

in welcher $R^1$, $R^2$, A und X die oben angegebenen Bedeutungen haben, mit Azolen der allgemeinen Formel (VI)

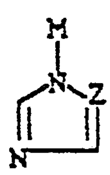

(VI)

in welcher
Z für eine Stickstoffatom oder die CH-Gruppe steht und
M für Wasserstoff oder Alkalimetall steht,
in Gegenwart eines geeigneten inerten organischen Lösungsmittels und gegebenenfalls in Gegenwart eines Säurebindemittel umsetzt;
und gegebenenfalls an die so erhaltenen Verbindungen der Formel (I) noch anschließend eine Säure oder ein Metallsalz addiert.

Die neuen heterocyclischen Amid-Derivate der Formel (I) sowie deren Säureadditions-Salze und Metallsalz-Komplexe weisen starke fungizide Eigenschaften auf. Dabei zeigen überraschenderweise die erfindungsgemäßen Verbindungen eine höhere Wirkung als die aus dem Stand der Technik bekannten N-[2-(2,4,6-Trichlorphenoxy)-ethyl]-N-propyl-1H-imidazol-1-carboxamid und N-Trichlormethylmercaptotetrahydrophtalimid, welche konstitutionell bzw. wirkungsmäßig naheliegende Verbindungen sind. Die erfindungsgemäßen Stoffen stellen somit eine Bereicherung der Technik dar.

Die erfindungsgemäßen heterocyclischen Amid-Derivate sind durch die Formel (I) allgemein definiert.

Besonders bevorzugt sind diejenigen Verbindungen der Formel (I), in denen

$R^1$ für geradkettiges oder verzweigtes Alkyl mit 1 bis 10 Kohlenstoffatomen, für gegebenenfalls einfach bis dreifach, gleich oder verschieden durch Fluor oder Chlor substituiertes Alkenyl oder Alkinyl mit jeweils 3 bis 10 Kohlenstoffatomen, wobei die Mehrfachbindung jeweils nicht in α-Stellung zum Stickstoffatom stehen darf, sowie für Alkoxyalkyl oder Alkylthioalkyl mit jeweils 1 bis 4 Kohlenstoffatomen im Alkoxyteil bzw. Alkylthioteil und 2 bis 4 Kohlenstoffatomen im Alkylteil, steht;

$R^2$ für gegebenenfalls einfach oder zweifach, gleich oder verschieden substituiertes Naphtyl, Tetrahydronaphthyl oder Indanyl steht, wobei als Substituenten jeweils Fluor, Chlor, Brom und Trifluormethoxy genannt seien;

A für eine geradkettige oder verzweigte Alkylenbrücke mit 1 bis 6 Kohlenstoffatomen steht, wenn x eine direkte Bindung bedeutet; bzw. für eine geradkettige oder verzweigte Alkylenbrücke mit 2 bis 6 Kohlenstoffatomen steht, wenn X Sauerstoff oder Schwefel bedeutet, wobei jedoch zwischen dem Stickstoffatom und dem Rest X mindestens 2 Kohlenstoffatome stehen müssen, oder für eine Alkylenbrücke mit 3 bis 5 Kohlenstoffatomen steht, wobei die Doppelbindung nicht in α-Stellung zum Stickstoffatom stehen darf;

X für Sauerstoff, Schwefel sowie eine direkte Bindung steht und

B für Imidazol-1-yl, Pyrazin-2-yl oder Pyridin-3-yl steht.

Ganz besonders bevorzugt sind diejenigen Verbindungen der Formel (I), in denen

$R^1$ für geradkettiges oder vezweigtes Alkyl mit 2 bis 8 Kohlenstoffatomen, geradkettiges oder verzweigtes Alkenyl oder Alkinyl mit jeweils 3 bis 8 Kohlenstoffatomen, wobei die Mehrfachbindung jeweils nicht in α-Stellung zum Stickstoffatom stehen darf, sowie für Methoxyethyl, Ethoxyethyl, n-Propoxyethyl, Methylthioethyl, Ethylthioethyl oder n-Propylthioethyl steht;

$R^2$ für gegebenenfalls einfach oder zweifach, gleich oder verschieden substituiertes Naphtyl, Tetrahydronaphthyl oder Indanyl steht, wobei als Substituenten Fluor, Chlor und Trifluormethoxy genannt seien;

A für eine geradkettige oder verzweigte Alkylenbrücke mit 1 bis 4 Kohlenstoffatomen steht, wenn X eine direkte Bindung bedeutet; bzw. für eine geradkettige oder verzweigte Alkylenbrücke mit 2 bis 4 Kohlenstoffatomen steht, wenn X Sauerstoff oder Schwefel bedeutet, wobei jedoch zwischen dem Stickstoffatom und dem Rest X mindestens 2 Kohlenstoffatome stehen müssen, oder für eine Alkenylbrücke mit 3 Kohlenstoffatomen steht, wobei die Doppelbindung nicht in α-Stellung zum Stickstoffatom stehen darf;

X für Sauerstoff, Schwefel sowie eine direkte Bindung steht und

B für Imidazol-1-yl oder Pyridin-3-yl steht.

Bevorzugte erfindungsgemäße Verbindungen sind auch Additionsprodukte aus Säuren und denjenigen heterocyclischen Amid-Derivaten der Formel (I), in denen die Substituenten $R^1$, $R^2$, A, X und B die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten heterocyclischen Amid-Derivaten der Formel (I), in denen die Substituenten $R^1$, $R^2$, A, X und B die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure; ferner Phosphorsäure, Salpetersäure und Schwefelsäure.

Im einzelnen seien außer den bei den Herstellungsbeispielen genannten Verbindungen die folgenden Verbindungen der allgemeinen Formel (I) genannt:

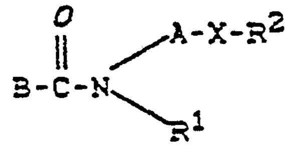

(I)

| $R^1$ | $R^2$ | A | X | B |
|---|---|---|---|---|
| $-CH_2CH_2CH_3$ | 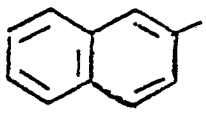 | $-CH=CHCH_2-$ | - | 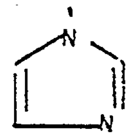 |
| $-CH_2CH_2CH_3$ | 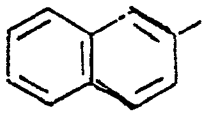 | $-CH_2CH_2CH_2-$ | - | 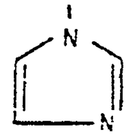 |
| $-CH_2CH_2CH_3$ | 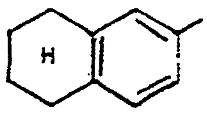 | $-CH_2CH_2-$ | O | 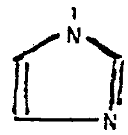 |
| $-CH_2-CH=CH_2$ | 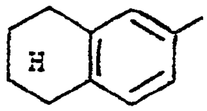 | $-CH_2CH_2-$ | O | 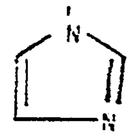 |
| $-CH_2CH_2CH_3$ | 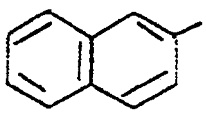 | $-CH_2CH_2-$ | O | 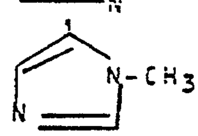 |
| $-CH_2CH_2CH_3$ | 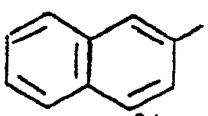 | $-CH_2CH_2-$ | O | 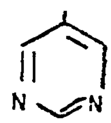 |
| $-CH_2CH_2CH_3$ | 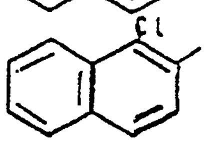 | $-CH_2CH_2-$ | O | 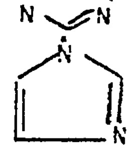 |
| $-CH_2CH_2CH_3$ | 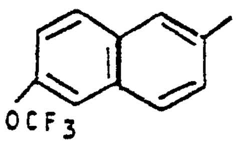 | $-CH_2CH_2-$ | O | 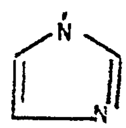 |
| $-CH_2CH_2CH_3$ | 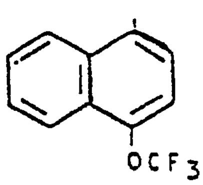 | $-CH_2CH_2-$ | O | 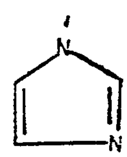 |
| $-CH_2-C\equiv CH$ | 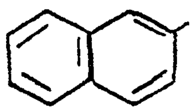 | $-CH_2-$ | - | 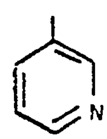 |

Verwendet man beispielsweise N-Methyl-N-(2-naphthylmethyl)-amin und N,N'-Carbonyldiimidazol als Ausgangsstoffe, so kann der Ablauf des erfingungsgemäßen Verfahrens (a) durch das folgende Formelschema wiedergegeben werden:

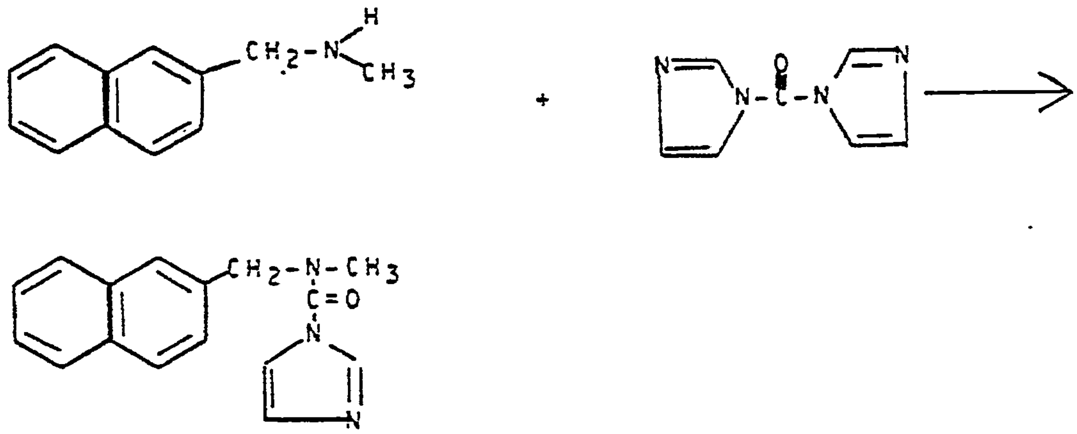

Setzt man anstatt N,N'-Carbonyldiimidazol das Pyrazin-2-carbon-imidazolid ein, so lautet die Reaktionsgleichung wie folgt:

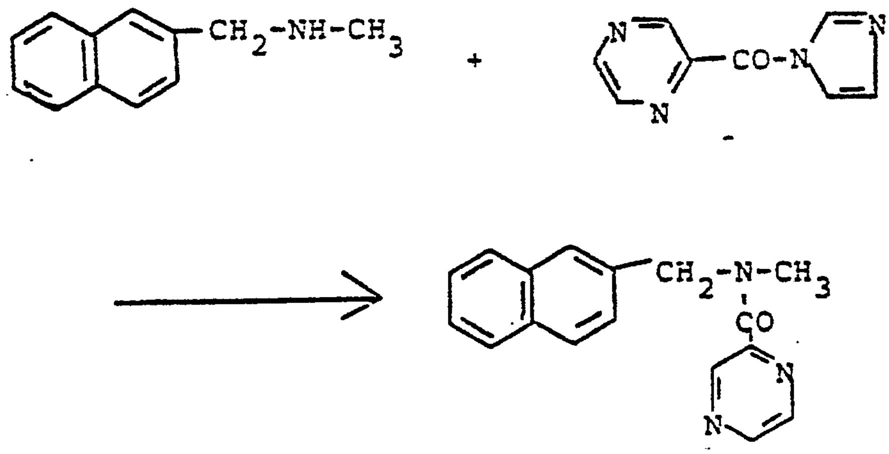

Verwendet man beispielsweise N-Methyl-N-(2-naphthylmethyl)-amin und Pyridin-3-ylcarbamoylchlorid als Ausgangsstoffe, so kann der Ablauf des erfingungsgemäßen Verfahrens (b) durch das folgende Formelschema wiedergegeben werden:

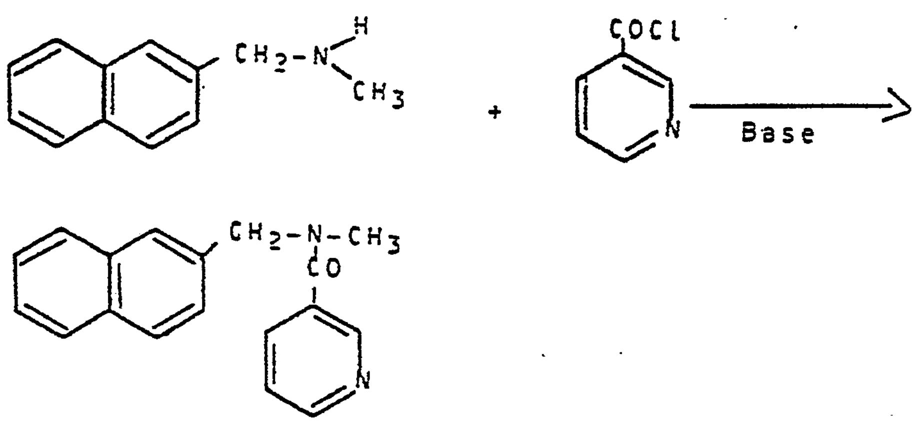


Verwendet man beispielsweise [N-Methyl-N-(2-naphthylmethyl)]-carbamoylchlorid und Imidazol als Ausgangsstoffe, so kann der Ablauf des erfingungsgemäßen Verfahrens (c) durch das folgende Formelschema wiedergegeben werden:

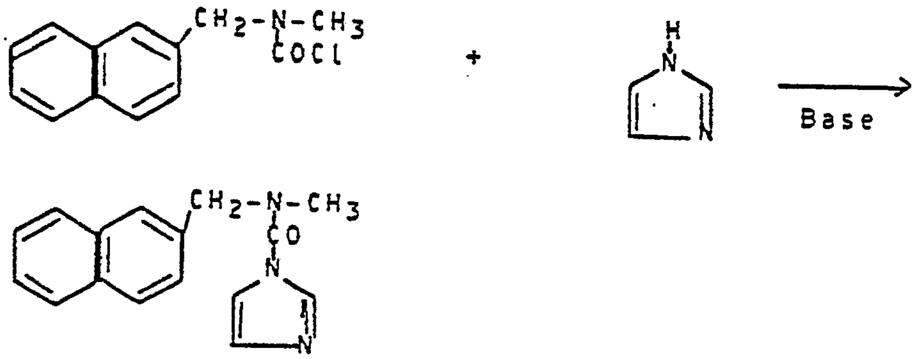


Die für die Durchführung des erfindungsgemäßen Verfahrens (a) und (b) als Ausgangsstoffe zu verwendenden Amine sind durch die Formel (II) allgemein definiert. In dieser Formel haben $R^1$, $R^2$, A und X vorzugsweise die Bedeutung, die bereits im Zusammenhang mit der Beschreibung der erfindungsgemäßen Stoffe der Formel (I) vorzugsweise für diese Substituenten genannt wurden.

Die Amine der Formel (II) sind teilweise bekannt (vgl. z.B. Helv. Chim. Acta 62, 1268 (1979) oder Chem. Abstr. 80, 36 879p (1974)) und können beispielsweise erhalten werden, indem man die entsprechenden Amid-Derivate mit Lithiumaluminiumhydrid in Gegenwart von Tetrahydrofuran bei Rückflußtemperaturen umsetzt. Die Amid-Derivate werden erhalten, indem man die entsprechende Carbonsäure mit Thionylchlorid unter Rückfluß versetzt und das so erhaltene Carbonylsäurechlorid mit dem entsprechenden Amin in Gegenwart von Tetrahydrofuran bei Raumtemperatur versetzt (vergleiche auch die Herstellungsbeispiele).

Die außerdem für die Durchführung des erfingungsgemäßen Verfahrens (a) als Ausgangsstoffe zu verwendenden Carbonyl-Verbindungen sind durch die Formeln (IIIa) und (IIIb) allgemein definiert. In dieser Formel (IIIa) hat B' vorzugsweise die oben genannten Bedeutungen.

Die Carbonyl-Verbindungen der Formeln (IIIa) und (IIIb) sind bekannt (vgl. z.B. Chem. Ber. 95, 1275 (1962) und Liebigs Annalen der Chemie 609, 75 (1957)). Die Verbindung der Formel (IIIb) können erhalten werden, indem man Triazol mit Phosgen in Gegenwart von Tetrahydrofuran bei Raumtemperatur umsetzt; die Verbindungen der Formel (IIIa) können erhalten werden, indem man die entsprechende Carbonsäure mit N,N'-Carbonyldiimidazol in Gegenwart von Tetrahydrofuran unter leichtem Erwärmen umsetzt. Die dazu benötigten Carbonsäuren sind allgemein bekannt. So kann 5-Pyridincarbonsäure erhalten werden, indem man 5-Brompyridin zuerst mit n-Butyllithium bei −110°C in Gegenwart von beispielsweise absolutem Ether oder Tetrahydrofuran umsetzt und anschließend festes Kohlendioxid hinzufügt (J. Org. Chem. 27, 2264 (1962)). 1-Methyl-5-imidazolcarbonsäure kann erhalten werden, indem man 1-Methyl-4,5-imidazoldicarbonsäure mit Essigsäureanhydrid bei erhöhter Temperatur umsetzt (vgl. Bull. Chem. Soc. Japan 53, 557 (1980)). 1-Methyl-4,5-imidazoldicarbonsäure kann erhalten werden, indem man 4,5-Imidazoldicarbonsäurediethylester mit Methyliodid und Natriummethylat in Gegenwart von Ethanol bei Rückflußtemperatur umsetzt und anschließend verseift (vgl. Journal of Heterocyclic Chemistry 1, 275 (1964)).

Die außerdem für die Durchführung des erfindungsgemäßen Verfahrens (b) als Ausgangsstoffe zu verwendenden Carbamoylchlorid-Verbindungen sind durch die Formel (IV) allgemein definiert. In dieser Formel hat B" vorzugsweise die oben genannte Bedeutung.

Die Carbamoylchlorid-Verbindungen der Formel (IV) sind bekannt (vgl. z.B. Liebigs Annalen der Chemie 766, 73 (1972); Bull. Chem. Soc. Japan 53, 557 (1980); Rec. trav. chim. 80, 1372 (1961)) bzw. können sie nach den dort beschriebenen Verfahren erhalten werden, indem man die entsprechenden Carbonsäuren mit Thionylchlorid unter Rückfluß versetzt (vgl. auch die Herstellungsbeispiele).

Die für die Durchführung des erfingungsgemäßen Verfahrens (c) als Ausgangsstoffe zu verwendenden Carbamoylchloride sind durch die Formel (V) allgemein definiert. In dieser Formel haben $R^1$, $R^2$, A und X vorzugsweise die Bedeutung, die bereits im Zusammenhang mit der Beschreibung der erfingungsgemäßen Stoffe der Formel (I) vorzugsweise für diese Substituenten genannt wurden.

Die Carbamoylchloride der Formel (V) lassen sich in allgemein bekannter Weise erhalten, indem man Amine der Formel (II) mit Phosgen in Gegenwart eines geeigneten inerten organischen Lösungsmittels, wie beispielsweise Acetonitril, Essigester, Toluol, Dichlormethan oder Dioxan, bei Temperaturen zwischen 20°C und 140°C umsetzt.

Die außerdem für die Durchführung des erfingungsgemäßen Verfahrens (c) als Ausgangsstoffe zu verwendenden Azole sind durch die Formel (VI) allgemein definiert. In dieser Formel steht Z für ein Stickstoffatom oder die CH-Gruppe. M steht vorzugsweise für Wasserstoff, Natrium oder Kalium.

Die Azole der Formel (VI) sind allgemein bekannte Verbindungen der organischen Chemie.

Das erfindungsgemäße Verfahren (a) wird in Gegenwart eines inerten organischen Lösungsmittels durchgeführt. Hierzu gehören vorzugsweise Nitrile, wie Acetonitril; Ester, wie Essigester; Ether, wie Dioxan; aromatische Kohlenwasserstoffe, wie Toluol; chlorierte Kohlenwasserstoffe, wie Dichlormethan; Säureamide, wie Dimethylformamid; und Sulfoxide, wie Dimethylsulfoxid. Vorzugsweise wird das Lösungsmittel absolut eingesetzt.

Die Reaktionstemperaturen können bei der Durchführung des erfingungsgemäßen Verfahrens (a) in einem größeren Bereich variiert werden. Im allgemeinen arbeitet man bei Temperaturen zwischen 20°C und 140°C, vorzugsweise zwischen 50°C und 120°C.

Die Umsetzung gemäß dem erfingungsgemäßen Verfahrens (a) kann bei Normaldruck, aber auch bei erhöhtem Druck durchgeführt werden. Im allgemeinen arbeitet man bei Drucken zwischen 1 und 50 bar, vorzugsweise zwischen 1 und 25 bar.

Das erfingungsgemäße Verfahren (b) wird in Gegenwart eines inerten organischen Lösungsmittels durchgeführt. Hierzu gehören vorzugsweise Nitrile, wie Acetonitril; Ester wie Essigester; Ether, wie Dioxan; aromatische Kohlenwasserstoffe, wie Toluol und chlorierte Kohlenwasserstoffe, wie Dichlormethan.

Als Säurebinder kommen für das erfindungsgemäße Verfahren (b) alle üblicherweise verwendbaren Basen infrage. Hierzu gehören vorzugsweise tertiäre Amine, wie Triethylamin oder Pyridin; Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 120°C, vorzugsweise bei Temperaturen zwischen 10°C und 100°C.

Das erfindungsgemäße Verfahren (c) wird in Gegenwart eines inerten organischen Lösungsmittels durchgeführt. Hierzu gehören vorzugsweise Nitrile, wie Acetonitril; Ester, wie Essigester; Ether wie Dioxan; aromatsiche Kohlenwasserstoffe, wie Toluol und chlorierte Kohlenwasserstoffe, wie Dichlormethan.

Als Säurebinder kommen für das erfindungsgemäße Verfahren (c) alle üblicherweise verwendbaren Basen in Frage. Hierzu gehören vorzugsweise tertiäre Amine, wie Triethylamin oder Pyridin, sowie ein Überschuß an Imidazol bzw. Triazol.

Die Reaktionstemperaturen können bei der Durchführung des erfindungsgemäßen Verfahrens (c) in einem größeren Bereich variiert werden. Im allgemeinen arbeitet man bei Temperaturen zwischen 20°C und 150°C, vorzugsweise bei Temperaturen zwischen 50°C und 120°C.

Die Umsetzung gemäß dem erfingungsgemäßen Verfahren (c) kann bei Normaldruck, aber auch bei erhöhtem Druck durchgeführt werden. Im allgemeinen arbeitet man bei Drucken zwischen 1 und 50 bar, vorzugsweise zwischen 1 und 25 bar.

Das erfindungsgemäße Verfahren (c) wird in Gegenwart eines inerten absoluten organischen Lösungsmittels durchgeführt. Hierzu gehören vorzugsweise Nitrile, wie Acetonitirl; Ether, wie Dioxan; aromatische Kohlenwasserstoffe, wie Toluol und chlorierte Kohlenwasserstoffe, wie Dichlormethan.

Zur Herstellung von Säureadditions-Salzen der Verbindungen der allgemeinen Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;

Sphaerotheca-Arten, wie beispielsweise Sphaerotheca fuliginea;
Podosphaera-Arten, wie beispielsweise Podosphaera leucotricha;
Venturia-Arten, wie beispielsweise venturia inaequalis;
Pyrenophora-Arten, wie beispielsweise Pyrenophora teres oder P. graminea;
(Konidienform: Drechslera, Syn: Helminthosporium);
Cochliobolus-Arten, wie beispielsweise Cochliobolus sativus;
(Konidienform: Drechslera, Syn: Helminthosporium );
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilage avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Venturia-Arten, wie Venturia inaequalis am Apfel und Puccinia-Arten, wie Puccinia recondita am Weizen sowie auch zur Bekämpfung von echtem Mehltau, Botrytis cinerea, Cochliobolus sativus, Pyrenophora teres und Fusarium an Getreide und von Pyricularia am Reis eingesetzt werden. Hervorzuheben ist die gute in vitro-Wirkung gegen Pyricularia oryzae am Reis.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorehtylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kiseslsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyehtylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele:

Beispiel 1

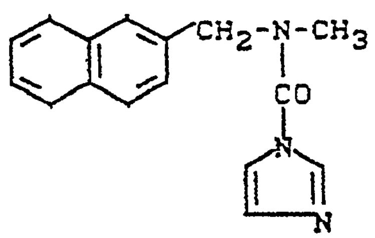

(Verfahren a)

Eine Lösung von 20 g (0,12 Mol) N-Methyl-N-(2-naphthylmethyl)-amin und 21,1 g (0,13 Mol) N,N'-Carbonyldiimidazol in 150 ml absoluten Acetonitril wird 4 Stunden unter Rückfluß erhitzt. Nach Entfernung des Lösungsmittels wird der Rückstand in Essigester aufgenommen, dreimal mit Wasser gewaschen, getrocknet und eingeengt.

Man erhält 30,9 g (das sind 97% der Theorie) [N-Methyl-N-(2-naphthylmethyl)]-1H-imidazol-1-carboxamid vom Schmelzpunkt 94,5–95,5°C.

Herstellung der Ausgangsprodukte:

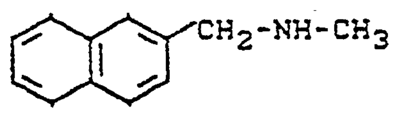

Eine Lösung von 105,2 g (0,57 Mol) [N-Methyl]-naphthalin-2-carboxamid in 450 ml absolutem Tetrahydrofuran wird in eine Suspension von 26,5 g (0,7 Mol) Lithiumaluminiumhydrid in 450 ml absolutem Ether eingetropft und anschließend 3 Stunden unter Rückfluß erhitzt.

Nach Aufarbeitung erhält man 96 g (98% der Theorie) N-Methyl-N-(2-naphthyl)-methylamin als hellbraunes Öl von 98% Reinheit.

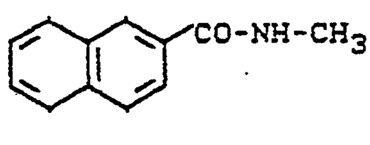

Eine Lösung von 100 g (0,58 Mol) Naphthalin-2-carbonsäure in 220 ml Thionylchlorid wird 1 Stunde unter Rückfluß erhitzt und anschließend das überschüssige Thionylchlorid abdestilliert. Der Rückstand wird in 750 ml Tetrahydrofuran suspendiert und unter Kühlung und Rühren mit 91 g (0,9 Mol) einer 30%igen wäßrigen Methylaminlösung versetzt. Anschließend werden 200 ml Triethylamin schnell eingetropft. Das Gemisch wird über Nacht bei Raumtemperatur gerührt. Nach Entfernen des Lösungsmittels wird der Rückstand zwischen Dichlormethan und Wasser verteilt.

Man erhält 105,2 g (98% der Theorie) [N-Methyl]-naphthalin-2-carboxamid vom Schmelzpunkt 108–109°C.

Beispiel 2

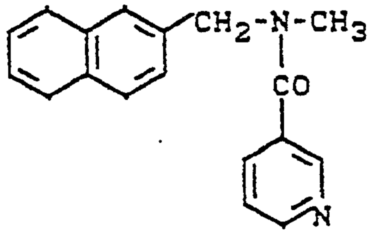

(Verfahren b)

Eine Lösung von 31 g (0,25 Mol) 3-Pyridincarbonsäure in 120 ml Thionylchlorid wird 1 Stunde unter Rückfluß erhitzt und anschließend das überschüssige Thionylchlorid unter Vakuum abdestilliert. Der Rückstand wird in 400 ml absolutem Tetrahydrofuran suspendiert und tropfenweise unter Rühren bei Raumtemperatur mit 30 g (0,18 Mol) N-Methyl-N-(2-naphthyl)-methylamin versetzt. 64,3 g (0,625 Mol) Triethylamin werden anschließend eingetropft und das Reaktionsgemisch über Nacht bei Raumtemperatur gerührt. Das Tetrahydrofuran wird entfernt, der Rückstand zwischen Methylenchlorid und Wasser verteilt, die Methylenchloridphase abgetrennt, einmal mit einer gesättigten Natriumhydrogencarbonatlösung und anschließend zweimal mit Wasser gewaschen, getrocknet und eingeengt.

Man erhält 42,7 g (86% der Theorie) [N-Methyl-N-(2-naphthyl)-methyl]-3-pyridincarboxamid als zähes Öl.

NMR ε ($CDCl_3$) 8,9–8,5 und 7,9–7,0 (11H); 4,87 und 4,65 (2H, 2s, breit); 3,07 und 2,90 (3H, 2s, breit).

In analoger Weise und entsprechend den erfindungsgemäßen Verfahren werden die nachfolgenden Verbindungen der allgemeinen Formel (I)

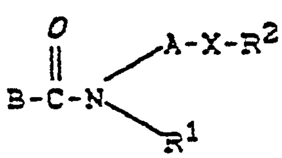

(I)

erhalten:

| Lfd Nr. | $R^1$ | $R^2$ | A | X | B | Schmelzpunkt (°C) bzw. spektroskopische Daten |
|---|---|---|---|---|---|---|
| 3 | $-CH_2CH_2CH_3$ | 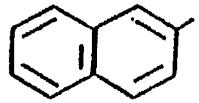 | $-CH_2CH_2-$ | 0 | 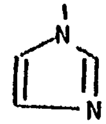 | 54 - 56 |
| 4 | $-CH_3$ | 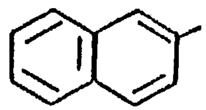 | $-CH_2CH_2-$ | 0 | 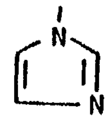 | 96,5 |
| 5 | $-CH_2CH_3$ | 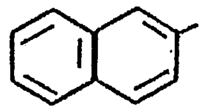 | $-CH_2CH_2-$ | 0 | 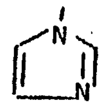 | 87-88 |
| 6 | $-CH_2CH=CH_2$ | 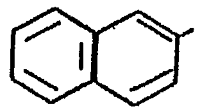 | $-CH_2CH_2-$ | 0 | 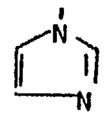 | 80 |
| 7 | $-CH_2CH_2CH_2CH_3$ | 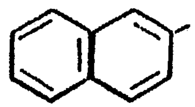 | $-CH_2CH_2-$ | 0 | 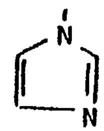 | Harz/ $^1H$-NMR $CDCl_3$: 3.46 (2H,t) für N $CH_2$ $CH_2CH_2CH_3$; 3.80(2H,t) für $O-CH_2-/CH_2/-N$ |
| 8 | $-CH_2CH_2CH_3$ | 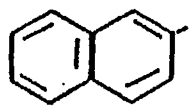 | $-CH_2-$ | – | 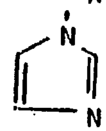 | Harz/ $^1H$-NMR $CDCl_3$: $\delta$ 4,78 (2H,s) für Naphthyl-$CH_2$-N; $\delta$ 3,36 (2H,t) für N-$CH_2$-$CH_2$ |
| 9 | $-CH_2CH_2CH_3$ | 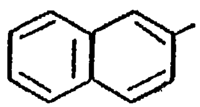 | $-CH_2CH_2-$ | 0 | 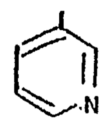 | Harz/ $^1H$-NMR $CDCl_3$: 3.20-3.65(2H,'s'breit) für N-$CH_2$ $CH_2CH_3$; 3.58-4.12 (2H,'s',breit) für $OCH_2$ $CH_2$ N |

| Lfd Nr. | $R^1$ | $R^2$ | A | X | B | Schmelzpunkt (°C) bzw. spektroskopische Daten |
|---|---|---|---|---|---|---|
| 10 | $-CH_2CH_2CH_3$ | 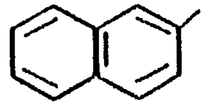 | $-CH_2CH_2-$ | O | 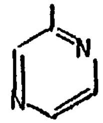 | |
| 11 | $-CH_2CH_2CH_3$ | 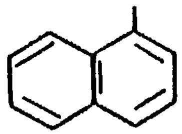 | $-CH_2CH_2-$ | O | 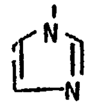 | 63,5 |
| 12 | $-CH_2CH_2CH_3$ | 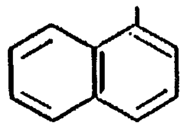 | $-CH_2CH_2-$ | O | 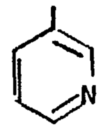 | Öl |
| 13 | $-CH_2CH_2CH_3$ | 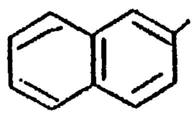 | $-CH_2CH_2-$ | - | 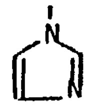 | $^1H$-NMR, $CDCl_3$: $\delta$ 3,27 (2H, t, $\tau$ =5Hz) für N-/$CH_2$/$CH_2CH_3$; $\delta$ 3,05 (2H, t, =5Hz) für Naphthyl-$CH_2$-/$CH_2$/-N |
| 14 | $-CH_2CH_2CH_3$ | 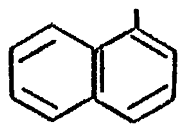 | $-CH_2CH_2-$ | - | 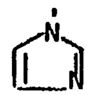 | 56-57 |
| 15 | $-CH_2CH_2OCH_3$ | 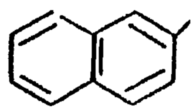 | $-CH_2CH_2-$ | O | 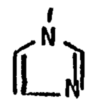 | |
| 16 | $-CH_2CH_2CH_3$ | 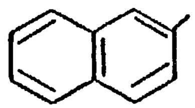 | $-CH_2CH_2-$ | S | 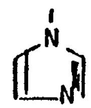 | |

| Lfd. Nr. | $R^1$ | $R^2$ | A | X | B | Schmelzpunkt (°C) bzw. spektroskopische Daten |
|---|---|---|---|---|---|---|
| 17 | $-CH_2CH_2CH_3$ | 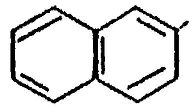 | $-CH_2CH_2-$ | O | 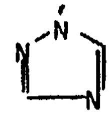 | |
| 18 | $-CH_2CH_3$ | 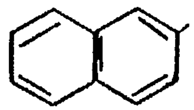 | $-CH_2-$ | – | 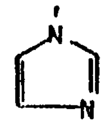 | Oel / $^1$H-NMR $CDCl_3$: δ 4,08 (2H,s) für Naphthyl-$CH_2$-N = δ 3,48 (2H,g) für N-$CH_2$-$CH_3$ |
| 19 | $-CH_2CH_2CH_2CH_3$ | 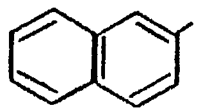 | $-CH_2-$ | – | 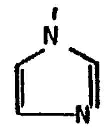 | Oel / $^1$H-NMR $CDCl_3$: δ 4,78 (2H,s) für Naphthyl-$CH_2$-N; = δ 3,39 (2H,t) für N-$CH_2$-$CH_2$ |
| 20 | $-CH_2CH=CH_2$ | 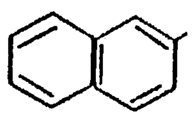 | $-CH_2-$ | – | 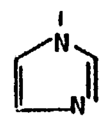 | Harz / $^1$H-NMR $CDCl_3$: δ 4,77 (2H,s) für Naphthyl-$CH_2$-N; δ 3,96 (2H,d) für N-$CH_2$-CH= |

| Lfd. Nr. | $R^1$ | $R^2$ | A | X | B | Schmelzpunkt (°C) bzw. spektroskopische Daten |
|---|---|---|---|---|---|---|
| 21 | $-CH_2CH_3$ | 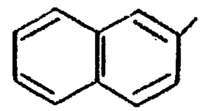 | $-CH_2-$ | - | 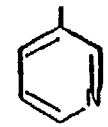 | Harz/ $^1$H-NMR $CDCl_3$: δ 5,08-4,50 (2H, s,breit) für Naphthyl-$CH_2$-N; δ 3,85-2,90 (2H, s) für N-$CH_2$-$CH_3$ |
| 22 | $-CH_2CH_2CH_3$ | 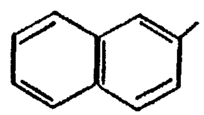 | $-CH_2-$ | - | 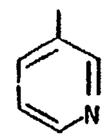 | Harz/ $^1$-NMR $CDCl_3$: δ 5,08-4,50, (2H, d, breit) für Naphthyl-$CH_2$-N; δ 3,70-2,97 (2H, d, breit) für N-$CH_2$-$CH_2$ |
| 23 | $-CH_2CH_2CH_2CH_3$ | 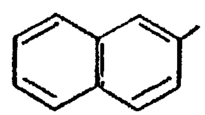 | $-CH_2-$ | - | 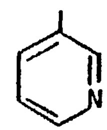 | Harz/ $^1$H-NMR $CDCl_3$: δ 5,08-4,50 (2H, d,breit) für Naphthyl-$CH_2$-N ; δ 3,75-2,95 (2H, d, breit) für N-$CH_2$-$CH_2$ |

| Lfd. Nr. | $R^1$ | $R^2$ | A | X | B | Schmelzpunkt(°C) bzw. spektroskopische Daten |
|---|---|---|---|---|---|---|
| 24 | $-CH_2CH=CH_2$ | 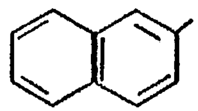 | $-CH_2-$ | - | 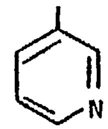 | Harz/ $^1H$-NMR $CDCl_3$: δ 5,08-4,50 (2H, s,breit) für Naphthyl-$CH_2$-N; δ 4,30-3,60 (2H,d,breit) für N-$CH_2$-CH= |

| Lfd. Nr. | $R^1$ | $R^2$ | A | X | B | Schmelzpunkt (°C) bzw. spektroskopische Daten |
|---|---|---|---|---|---|---|
| 25 | $-CH_2-CH=CH_2$ | 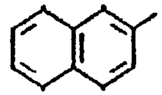 | $-CH_2-CH_2-$ | O | 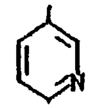 | Öl |
| 26 | $-CH_3$ | 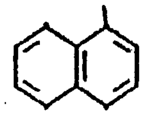 | $-CH_2-CH_2-$ | O | 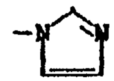 | 81,5-82,5 |
| 27 | $-CH_3$ | 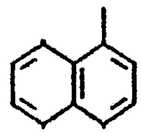 | $-CH_2-CH_2-$ | O | 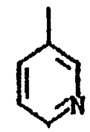 | Öl |
| 28 | $-CH_2-CH_3$ | 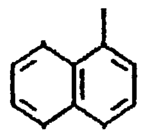 | $-CH_2-CH_2-$ | O | 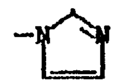 | 92,5 |
| 29 | $-CH_2-CH_3$ | 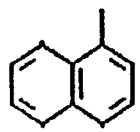 | $-CH_2-CH_2-$ | O | 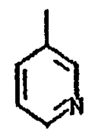 | Öl |
| 30 | $-(CH_2)_3-CH_3$ | 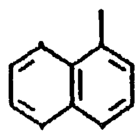 | $-CH_2-CH_2-$ | O | 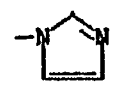 | 87,5 |
| 31 | $-(CH_2)_3-CH_3$ | 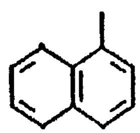 | $-CH_2-CH_2$ | O | 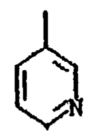 | Öl |

| Lfd. Nr. | $R^1$ | $R^2$ | A | X | B | Schmelzpunkt (°C) bzw. spektroskopische Daten |
|---|---|---|---|---|---|---|
| 32 | $-CH_2-CH=CH_2$ | 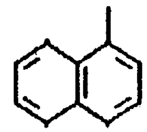 | $-CH_2-CH_2-$ | O | 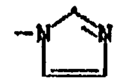 | 80-81 |
| 33 | $-(CH_2)_3-CH_3$ | 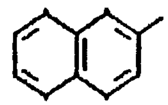 | $-CH_2-CH_2$ | - | 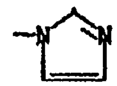 | $^1H$-NMR $CDCl_3$:<br>δ 3,30 (2H,t,τ=5Hz) für N-[$CH_2$]-$CH_2$-$CH_2$-$CH_3$<br>δ 3,05 (2H,t,τ=5Hz), für Naphthyl-$CH_2$-[$CH_2$]-N |
| 34 | $-(CH_2)_3-CH_3$ | 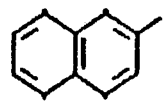 | $-CH_2-CH_2$ | - | 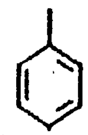 | $^1H$-NMR $CDCl_3$:<br>δ 3,96-3,38 (3H,'S',breit) und 3,38-2,78 (3H,'S',breit), für Naphthyl-[$CH_2$-$CH_2$]-N-[$CH_2$]-$CH_2$-$CH_2$-$CH_3$ |
| 35 | $-(CH_2)_2-CH_3$ | 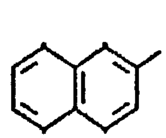 | $-CH_2-CH_2-$ | - | 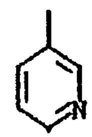 | $^1H$-NMR $CDCl_3$:<br>δ 3,94-3,36 (3H,'S',breit) und 3,36-2,73 (3H,'S',breit), für Naphthyl-[$CH_2$-$CH_2$]-N-[$CH_2$]-$CH_2$-$CH_3$ |
| 36 | $-CH_2-CH=CH_2$ | 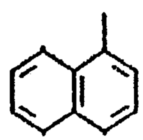 | $-CH_2-CH_2-$ | O | 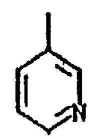 | $^1H$-NMR $CDCl_3$:<br>δ 4,27-3,88 (4H,'S',breit), für Naphthyl-O-$CH_2$-[$CH_2$]-N-[$CH_2$]-CH=$CH_2$ |
| 37 | $-CH_2-CH_3$ | 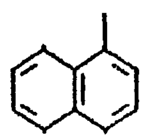 | $-CH_2-CH_2-$ | - | 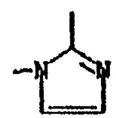 | $^1H$-NMR $CDCl_3$ (300 MHz):<br>δ 3,44 (2H,t), für Naphthyl-$CH_2$-[$CH_2$]-N-$C_2H_5$;<br>δ 3,37 (2H,q), für N-[$CH_2$]-$CH_3$ |

| Lfd. Nr. | $R^1$ | $R^2$ | A | X | B | Schmelzpunkt (°C) bzw. spektroskopische Daten |
|---|---|---|---|---|---|---|
| 38 | $-(CH_2)_3-CH_3$ | 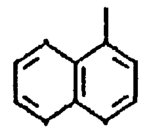 | $-CH_2-CH_2-$ | - | 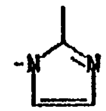 | $^1H$-NMR $CDCl_3$ (300 MHz): δ 3,41 (2H,t) für Naphthyl $CH_2-\boxed{CH_2}-N-C_4H_9$ δ 3,26 (2H,t), für $-N-\boxed{CH_2}-C_3H_7$ |
| 39 | $-(CH_2)_4-CH_3$ | 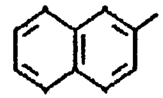 | $-CH_2-CH_2-$ | O | 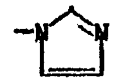 | |
| 40 | $-(CH_2)_5-CH_3$ | 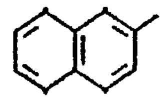 | $-CH_2-CH_2-$ | O | 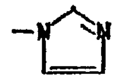 | |
| 41 | $-(CH_2)_6-CH_3$ | 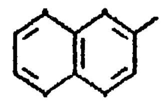 | $-CH_2-CH_2-$ | O | 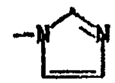 | |
| 42 | $-(CH_2)_4-CH_3$ | 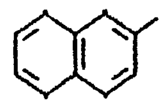 | $-CH_2-$ | - | 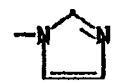 | |
| 43 | $-(CH_2)_5-CH_3$ | 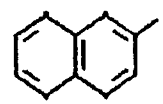 | $-CH_2-$ | - | 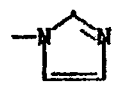 | |
| 44 | $-(CH_2)_6-CH_3$ | 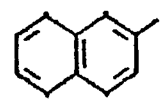 | $-CH_2-$ | - | 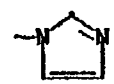 | |
| 45 | $-(CH_2)_2-CH_3$ | 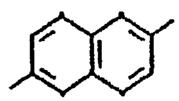 Br | $-CH_2-CH_2-$ | O | 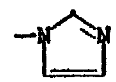 | 112-114 |

Verwendungsbeispiele:

In den nachfolgenden Verwendungsbeispielen werden die nachstehend angegebenen Substanzen als Vergleichsverbindungen eingesetzt.

(A) 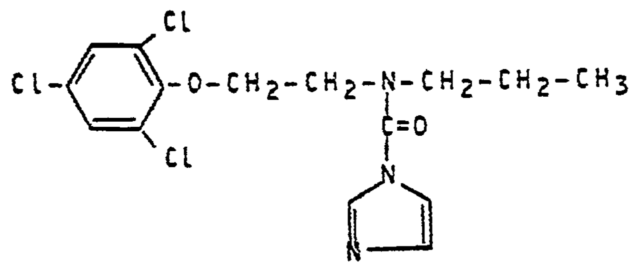

(B) 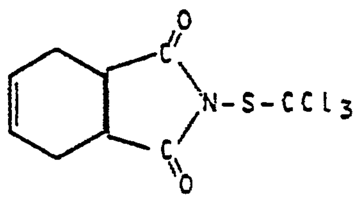

Beispiel A

Puccinia-Test (Weizen) / protektiv /
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit einer 0,1%igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 3.

Beispiel B

Venturia-Test (Apfel) / protektiv
Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% rel. Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 3.

## Patentansprüche

1. Heterocyclische Amid-Derivate der allgemeinen Formel (I)

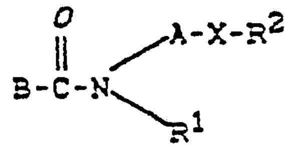

(I)

in welcher
$R^1$ für Alkyl mit 1 bis 12 Kohlenstoffatomen und für gegebenenfalls durch Fluor oder Chlor substituiertes Alkenyl und Alkinyl mit jeweils 3 bis 12 Kohlenstoffatomen steht, wobei die Mehrfachbindung jeweils nicht in α-Stellung zum Stickstoffatom stehen darf, sowie für Alkoxyalkyl oder Alkylthioalkyl mit jeweils 1 bis 6 Kohlenstoffatomen im Alkoxy- bzw. Alkylthioteil und 2 bis 6 Kohlenstoffatomen im Alkylteil steht;
$R^2$ für gegebenenfalls einfach bis dreifach, gleich oder verschieden durch Alkyl mit 1 bis 4 Kohlenstoffatomen, Halogen und Halogenalkoxy mit 1 bis 4 Kohlenstoffatomen und 1 bis 5 gleichen oder verschiedenen Halogenatomen substituiertes Naphthyl, Tetrahydronaphthyl oder Indanyl steht;
A für eine geradkettige oder verzweigte Alkylenbrücke mit 1 bis 8 Kohlenstoffatomen steht, wenn X eine direkte Bindung bedeutet, bzw. für eine geradkettige oder verzweigte Alkylenbrücke mit 2 bis 8 Kohlenstoffatomen steht, wenn X Sauerstoff oder Schwefel bedeutet, wobei jedoch zwischen dem Stickstoffatom und dem Rest X mindestens 2 Kohlenstoffatome stehen müssen, oder für eine geradkettige oder verzweigte Alkylenbrücke mit 3 bis 8 Kohlenstoffatomen steht, wobei die Doppelbindung nicht in α-Stellung zum Stickstoffatom stehen darf;
X für Sauerstoff, Schwefel, sowie für eine direkte Bindung steht und
B für Imidazol-1-yl, Pyridin-3-yl, Pyrazin-2-yl, Pyrimidin-5-yl, 1,2,4-Triazol-1-yl sowie für 1-Methylimidazol-5-yl steht,
sowie deren Säureadditions-Salze und Metallsalz-Komplexe.

2. Verfahren zur Herstellung von heterocyclischen Amid-Derivaten der allgemeinen Formel (I)

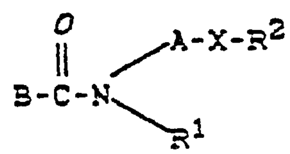

(I)

in welcher
$R^1$ für Alkyl mit 1 bis 12 Kohlenstoffatomen und für gegebenenfalls durch Fluor oder Chlor substituiertes Alkenyl und Alkinyl mit jeweils 3 bis 12 Kohlenstoffatomen steht, wobei die Mehrfachbindung jeweils nicht in α-Stellung zum Stickstoffatom stehen darf, sowie für Alkoxyalkyl oder Alkylthioalkyl mit jeweils 1 bis 6 Kohlenstoffatomen im Alkoxy- bzw. Alkylthioteil und 2 bis 6 Kohlenstoffatomen im Alkylteil steht;
$R^2$ für gegebenenfalls einfach bis dreifach, gleich oder verschieden durch Alkyl mit 1 bis 4 Kohlenstoffatomen, Halogen und Halogenalkoxy mit 1 bis 4 Kohlenstoffatomen und 1 bis 5 gleichen oder verschiedenen Halogenatomen substituiertes Naphthyl, Tetrahydronaphthyl oder Indanyl steht;
A für eine geradkettige oder verzweigte Alkylenbrücke mit 1 bis 8 Kohlenstoffatomen steht, wenn X eine direkte Bindung bedeutet, bzw. für eine geradkettige oder verzweigte Alkylenbrücke mit 2 bis 8 Kohlenstoffatomen steht, wenn X Sauerstoff oder Schwefel bedeutet, wobei jedoch zwischen dem Stickstoffatom und dem Rest X mindestens 2 Kohlenstoffatome setehen müssen, oder für eine geradkettige oder verzweigte Alkylenbrücke mit 3 bis 8 Kohlenstoffatomen steht, wobei die Doppelbindung nicht in α-Stellung zum Stickstoffatom stehen darf;
X für Sauerstoff, Schwefel, sowie für eine direkte Bindung steht und
B für Imidazol-1-yl, Pyridin-3-yl, Pyrazin-2-yl, Pyrimidin-5-yl, 1,2,4-Triazol-1-yl sowie für 1-Methylimidazol-5-yl steht,
sowie von deren Säureadditions-Salzen und Metallsalz-Komplexen, dadurch gekennzeichnet, daß man

a) Amine der allgemeinen Formel (II)

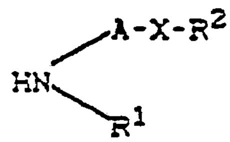

(II)

in welcher
$R^1$, $R^2$, A und X die oben angegebenen Bedeutungen haben, mit Carbony-Verbindungen der allgemeinen Formel (IIIa)

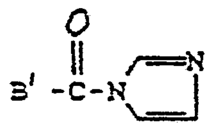

(IIIa)

in welcher
B' für Imidazolyl-1-yl, Pyridin-3-yl, Pyrazin- 2-yl, Pyrimidin- 5-yl oder 1-Methylimidazol-5-yl steht, oder mit einer Carbonyl-Verbindung der allgemeinen Formel (IIIb)

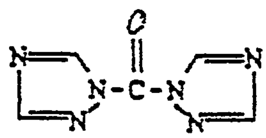

(IIIb)

in Gegenwart eines geeigneten inerten organischen Lösungsmittel umsetzt; oder
b) Amine der allgemeinen Formel (II)

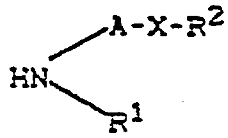

(II)

in welcher
$R^1$, $R^2$, A und X die oben angegebenen Bedeutungen haben, mit Carbamoylchlorid-Verbindungen der Formel (IV)

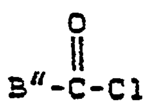

(IV)

in welcher
B" für Pyridin-3-yl, Pyrazin-2-yl, Pyrimidin-5- yl, oder 1-Methylimidazol-5-yl steht,
in Gegenwart eines geeigneten inerten organischen Lösungsmittels und in Gegenwart eines Säurebindemittels umsetzt; oder
c) Carbamoylchlorid–Derivate der allgemeinen Formel (V)

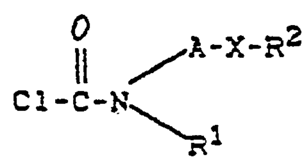

(V)

in welcher
$R^1$, $R^2$, A und X die oben angegebenen Bedeutungen haben, mit Azolen der allgemeinen Formel (VI)

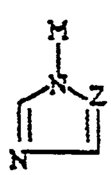

(VI)

in welcher
Z für ein Stickstoffatom oder die CH-Gruppe steht und
M für Wasserstoff oder Alkalimetall steht,
in Gegenwart eines geeigneten inerten organischen Lösungsmittels und gegebenfalls in Gegenwart eines Säurebindemittels umsetzt;
und gegebenenfalls an die so erhaltenen Verbindungen der Formel (I) noch anschließend eine Säure oder ein Metallsalz addiert.

3. Fungizide Mittel, gekennzeichnet durch einen Gehalt an mindestens einem heterocyclischen Amid-Derivat der Formel (I) in Anspruch 1 bzw. an einem Säureadditions-Salz oder Metallsalz-Komplex einer Verbindung der Formel (I).

4. Verfahren zur Bekämpfung von Pilzen im Pflanzenschutz, dadurch gekennzeichnet, daß man heterocyclische Amid-Derivate der Formel (I) in Anspruch 1 bzw. deren Säureadditions-Salze oder Metallsalz-Komplexe auf Pilze oder ihren Lebensraum einwirken läßt.

5. Verwendung von heterocyclischen Amid-Derivaten der Formel (I) in Anspruch 1 bzw. von deren Säureadditions-Salzen oder Metallsalz-Komplexen zur Bekämpfung von Pilzen im Pflanzenschutz.

6. Verfahren zur Herstellung von fungiziden Mitteln, dadurch gekennzeichnet, daß man heterocyclische Amid-Derivate der Formel (I) in Anspruch 1 bzw. deren Säureadditions-Salze oder Metallsalz-Komplexe mit Streckmitteln und/oder oberflächenaktiven Mitteln vermischt.

**Claims**

1. Heterocyclic amide derivatives of the general formula (I)

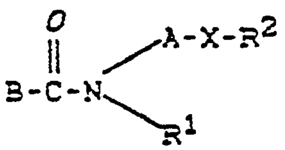

(I)

in which
$R^1$ represents alkyl which has 1 to 12 carbon atoms and represents alkenyl and alkinyl, each of which has 3 to 12 carbon atoms and each of which is optionally substituted by fluorine or chlorine, where the multiple bond in each case must not be in the α-position to the nitrogen atom, and also represents alkoxy-alkyl or alkylthioalkyl, each of which has 1 to 6 carbon atoms in the alkoxy or alkylthio moiety, respectively, and 2 to 6 carbon atoms in the alkyl moiety;
$R^2$ represents naphthyl, tetrahydronaphthyl or indanyl, each of which is optionally monosubstituted to trisubstituted by identical or different alkyl substituents which have 1 to 4 carbon atoms, halogen and halogenalkoxy which has 1 to 4 carbon atoms and 1 to 5 identical or different halogen atoms;
A represents a straight-chain or branched alkylene bridge which has 1 to 8 carbon atoms if X denotes a direct bond, or represents a straight-chain or branched alkylene bridge which has 2 to 8 carbon atoms if X denotes oxygen or sulphur but where at least 2 carbon atoms must stand between the nitrogen atom and the radical X, or represents a straight-chain or branched alkylene bridge which has 3 to 8 carbon atoms, where the double bond must not be in the α-position to the nitrogen atom;
X represents oxygen, sulphur, and also a direct bond and
B represents imidazol-1-yl, pyridin-3-yl, pyrazin-2-yl, pyrimidin-5-yl, 1,2,4-triazol-1-yl and also 1-methyl-imidazol-5-yl,
and the acid addition salts and metal salt complexes thereof.

2. Process for the preparation of heterocyclic amide derivatives of the general formula (I)

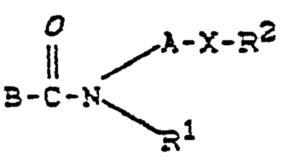

(I)

in which
$R^1$ represents alkyl which has 1 to 12 carbon atoms and represents alkenyl and alkinyl, each of which has 3 to 12 carbon atoms and each of which is optionally substituted by fluorine or chlorine, where the multiple bond in each case must not be in the α-position to the nitrogen atom, and also represents alkoxyalkyl or alkylthioalkyl, each of which has 1 to 6 carbon atoms in the alkoxy or alkylthio moiety, respectively, and 2 to 6 carbon atoms in the alkyl moiety;
$R^2$ represents naphthyl, tetrahydronaphthyl or indanyl, each of which is optionally monosubstituted to trisubstituted by identical or different alkyl substituents which have 1 to 4 carbon atoms, halogen and halogenalkoxy which has 1 to 4 carbon atoms and 1 to 5 identical or different halogen atoms;
A represents a straight-chain or branched alkylene bridge which has 1 to 8 carbon atoms if X denotes a direct bond, or represents a straight-chain or branched alkylene bridge which has 2 to 8 carbon atoms if X denotes oxygen or sulphur but where at least 2 carbon atoms must stand between the nitrogen atom and the radical X, or represents a straight-chain or branched alkylene bridge which has 3 to 8 carbon atoms, where the double bond must not be in the α-position to the nitrogen atom;
X represents oxygen, sulphur, and also a direct bond and
B represents imidazol-1-yl, pyridin-3-yl, pyrazin-2-yl, pyrimidin-5-yl, 1,2,4-triazol-1-yl and also 1-methyl-imidazol-5-yl,
and the acid addition salts and metal salt complexes thereof, characterized in that

a) amines of the general formula (II)

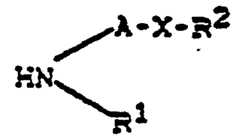

in which
$R^1$, $R^2$, A and X have the abovementioned meanings are reacted with carbonyl compounds of the general formula (IIIa)

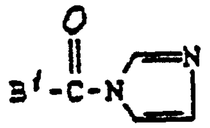

in which
B' represents imidazolyl-1-yl, pyridin-3-yl, pyrazin-2-yl, pyrimidin-5-yl or 1-methylimidazol-5-yl, or with a carbonyl compound of the general formula (IIIb)

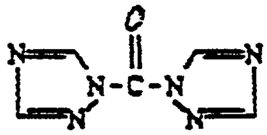

in the presence of a suitable inert organic solvent; or
b) amines of the general formula (II)

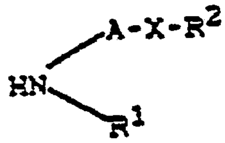

in which
$R^1$, $R^2$, A and X have the abovementioned meanings are reacted with carbamoyl chloride compounds of the formula (IV)

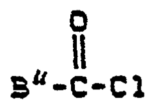

in which
B" represents pyridin-3-yl, pyrazin-2-yl, pyrimidin-5-yl, or 1-methylimidazol-5-yl, in the presence of a suitable inert organic solvent and in the presence of an acid-binding agent; or
c) carbamoyl chloride derivatives of the general formula (V)

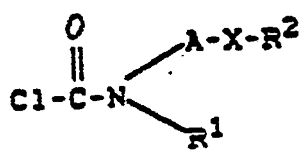

in which
$R^1$, $R^2$, A and X have the abovementioned meanings are reacted with azoles of the general formula (VI)

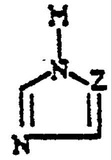

in which
Z represents a nitrogen atom or the CH group and
M represents hydrogen or alkali metal, in the presence of a suitable inert organic solvent and if

appropriate in the presence of an aid-binding agent;
and, if desired, an acid or a metal salt are subsequently added on to the resulting compounds of the formula (I).

3. Fungicidal agents, characterized in that they contain at least one heterocyclic amide derivative of the formula (I) in Claim 1 or an acid addition salt or metal salt complex of a compound of the formula (I).

4. Method of combating fungi in plant protection, characterized in that heterocyclic amide derivatives of the formula (I) in Claim 1 or acid addition salts or metal salt complexes thereof are allowed to act on fungi or their habitat.

5. Use of heterocyclic amide derivatives of the formula (I) in Claim 1 or of acid addition salts or metal salt complexes thereof for combating fungi in plant protection.

6. Process for the preparation of fungicidal agents, characterized in that heterocyclic amide derivatives of the formula (I) in Claim 1 or acid addition salts or metal salt complexes thereof are mixed with extenders and/or surface-active agents.

**Revendications**

1. Dérivés d'amides hétérocycliques de formule générale I

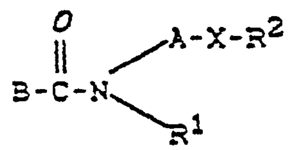

(I)

dans laquelle
$R^1$ représente un groupe alkyle en $C_1$–$C_{12}$ ou un groupe alcényle ou alcynyle contenant chacun 3 à 12 atomes de carbone et éventuellement substitués par le fluor ou le chlore, étant spécifié toutefois que la liaison multiple ne doit pas se trouver en position $\alpha$ de l'atome d'azote, ou un gorupe alcoxyalkyle ou alkylthioalkyle contenant chacun 1 à 6 atomes de carbone dans la partie alcoxy ou alkylthio respectivement, et 2 à 6 atomes de carbone dans la partie alkyle;
$R^2$ représente un groupe naphtyle, tétrahydronaphtyle ou indanyle portant éventuellement 1 à 3 substituants identiques ou différents choisis parmi les groupes alkyle en $C_1$–$C_4$, les halogènes et les groupes halogénoalcoxy contenant 1 à 4 atomes de carbone et 1 à 5 atomes d'halogènes identiques ou différents;
A représente un pont alkylène à chaîne droite ou ramifiée en $C_1$–$C_8$ lorsque X représente une liaison directe, un pont alkylène à chaîne droite ou ramifiée en $C_2$-$C_8$ lorsque X représente l'oxygène ou le soufre, étant spécifié toutefois qu'entre l'atome d'azote et l'atome X il doit y avoir au moins 2 atomes de carbone, ou bien un pont alkylène à chaîne droite ou ramifiée en $C_3$–$C_8$, étant spécifié que la double liaison ne doit pas se trouver en position $\alpha$ de l'atome d'azote;
X représente l'oxygène, le soufre ou une liaison directe, et
B représente un groupe imidazole-1-yle, pyridine-3-yle, pyrazine-2-yle, pyrimidine-5-yle, 1,2,4-triazole-1-yle ou 1-méthyl-imidazole-5-yle,
et leurs sels formés par addition avec des acides et complexes de sels métalliques.

2. Procédé de préparation des dérivés d'amides hétérocycliques de formule général I

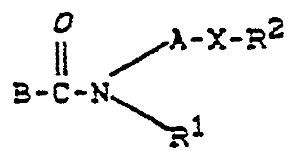

(I)

dans laquelle
$R^1$ représente un groupe alkyle en $C_1$–$C_{12}$ ou un groupe alcényle ou alcynyle contenant chacun 3 à 12 atomes de carbone éventuellement substitués par le fluor ou le chlore, étant spécifié toutefois que la liaison multiple ne doit pas se trouver en position $\alpha$ de l'atome d'azote, ou un groupe alcoxyalkyle ou alkylthioalkyle contenant chacun 1 à 6 atomes de carbone dans la partie respective alcoxy ou alkylthio et 2 à 6 atomes de carabone dans la partie alkyle;
$R^2$ représente un groupe naphtyle, tétrahydronaphtyle ou indanyle portant éventuellement 1 à 3 substituants identiques ou différents choisis parmi les groupes alkyle en $C_1$–$C_4$, les halogènes et les groupes halogénoalcoxy contenant 1 à 4 atomes de carbone et 1 à 5 atomes d'halogènes identiques ou différents;
A représente un pont alkylène à chaîne droite ou ramifiée en $C_1$–$C_8$ lorsque X représente une liaison directe, un point alkylène à chaîne droite ou ramifiée en $C_2$-$C_8$ lorsque X représente l'oxygène ou le soufre, étant spécifié toutefois qu'entre l'atome d'azote et l'atome X il doit y avoir au moins 2 atomes de carbone, ou bien un pont alkylène à chaîne droite ou ramifiée en $C_3$–$C_8$, étant spécifié que la double liaison ne doit pas se trouver en position $\alpha$ de l'atome d'azote;

X représente l'oxygène, le soufre ou une liaison directe, et
B représente un groupe imidazole-1-yle, pyridine-3-yle, pyrazine-2-yle, pyrimidine-5-yle, 1,2,4-triazole-1-yle ou 1-méthyl-imidazole-5-yle,
de leurs sels formés par addition avec des acides et de leurs complexes de sels métalliques, caractérisé en ce que:

a) on fait réagir des amines de formule générale II

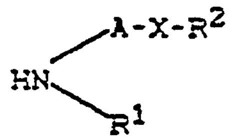

(II)

dans laquelle
$R^1$, $R^2$, A et X ont les significations indiquées ci-dessus, avec des composés carbonylés de formule générale IIIa

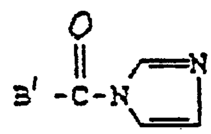

(IIIa)

dans laquelle
B' représente un groupe imidazole-1-yle, pyridine-3-yle, pyrazine-2-yle, pyrimidine-5-yle ou 1-méthyl-imidazole-5-yle, ou avec un composé carbonylé de formule générale IIIb

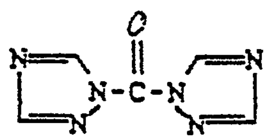

(IIIb)

en présence d'un solvant organique inerte approprié; ou bien

b) on fait réagir des amines de formule générale II

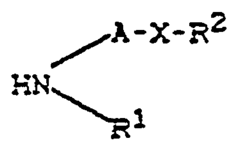

(II)

dans laquelle
$R^1$, $R^2$, A et X ont les significations indiquées ci-dessus,
avec des dérivés du chlorure de carbamoyle répondant à la formule IV

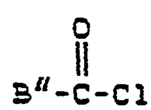

(IV)

dans laquelle
B" représente un groupe pyridine-3-yle, pyrazine-2-yle, pyrimidine-5-yle ou 1-méthylimidazole-5-yle, en présence d'un solvant organique inerte approprié et en présence d'un capteur d'acide; ou bien

c) on fait réagir des dérivés du chlorure de carbamoyle répondant à la formule générale V

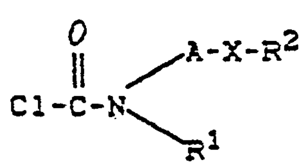

(V)

dans laquelle
$R^1$, $R^2$, A et X ont les significations indiquées ci-dessus, avec des azoles de formule générale VI

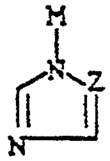

(VI)

dans laquelle
Z représente un atome d'azote ou le groupe CH,
M représente l'hydrogène ou un métal alcalin, en présence d'un solvant organique inerte approprié et le cas échéant en présence d'un capteur d'acide;
et le cas échéant, sur les composés de formule I ainsi obtenus, on fixe encore par addition un acide ou un sel métallique.

3. Produite fongicides, caractérisés en ce qu'ils contiennent au moins un dérivé d'amide hétérocyclique de formule I de la revendication 1 ou un sel formé par addition avec un acide ou un complexe de sel métallique d'un composé de formule I.

4. Procédé pour combattre les mycètes pour la protection des végétaux, caractérisé en ce que l'on fait agir les dérivés d'amides hétérocycliques de formule I de la revendication 1 ou leurs sels formés par addition avec des acides ou complexes de sels métalliques sur les mycètes ou leur habitat.

5. Utilisation des dérivés d'amides hétérocycliques de formule I de la revendication 1 ou de leurs sels formés par addition avec des acides ou complexes de sels métalliques dans la lutte contre les mycètes pour la protection des végétaux.

6. Procédé de préparation des produits fongicides, caractérisé en ce que l'on mélange des dérivés d'amides hétérocycliques de formule I de la revendication 1 ou leurs sels formés par addition avec des acides ou complexes de sels métalliques avec des diluants et/ou des agents tensioactifs.